# EUROPÄISCHE PATENTSCHRIFT

(11) **EP 2 729 800 B1**
(45) Veröffentlichungstag und Bekanntmachung des Hinweises auf die Patenterteilung: **03.10.2018**
(21) Anmeldenummer: 12743396.9
(22) Anmeldetag: 27.06.2012
(51) Int. Cl.: G01N 33/497

(54) **ÜBERWACHUNG DER FUNKTIONALITÄT EINES KONVERTERS EINES ATEMANALYSEGERÄTES**
MONITORING OF THE FUNCTIONALITY OF A CONVERTER OF A BREATH ANALYSIS APPARATUS
SURVEILLANCE DE LA FONCTIONNALITÉ D'UN CONVERTISSEUR D'UN ANALYSEUR D'HALEINE

(30) Priorität: 08.07.2011 DE 102011078867; 10.08.2011 DE 102011080765
(43) Veröffentlichungstag der Anmeldung: 14.05.2014
(73) Patentinhaber: Robert Bosch GmbH, 70442 Stuttgart (DE)
(72) Erfinder: FLEISCHER, Maximilian, 85635 Höhenkirchen (DE); MAGORI, Erhard, 85622 Feldkirchen (DE); POHLE, Roland, 85570 Herdweg (DE); REUTER, Florian, 80797 München (DE)
(86) Internationale Anmeldenummer: PCT/EP2012/062421
(87) Internationale Veröffentlichungsnummer: WO 2013/007516

(56) Entgegenhaltungen:
- WO-A1-2010/115694
- DE-A1- 4 308 894
- FRUHBERGER B ET AL: "Detection and quantification of nitric oxide in human breath using a semiconducting oxide based chemiresistive microsensor", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, Bd. 76, Nr. 1-3, 1. Juni 2001 (2001-06-01) , Seiten 226-234, XP004241122, ISSN: 0925-4005, DOI: 10.1016/S0925-4005(01)00572-X
- OLEKSANDR KUZMYCH ET AL: "Carbon nanotube sensors for exhaled breath components", NANOTECHNOLOGY, IOP, BRISTOL, GB, Bd. 18, Nr. 37, 19. September 2007 (2007-09-19), Seite 375502, XP020119566, ISSN: 0957-4484, DOI: 10.1088/0957-4484/18/37/375502

## Beschreibung

Überwachung der Funktionalität eines Konverters eines Atemanalysegerätes Die Messung von Markergasen in der menschlichen Ausatemluft stellt eine zukunftsträchtige nichtinvasive Technik dar, um Krankheiten und metabolische Fehlfunktionen zu erkennen. Mögliche Anwendungen betreffen hier sowohl Screening, Differentialdiagnose wie auch Therapieoptimierung. Beispiele dafür sind die NO-Detektion zur Verlaufskontrolle einer Asthmatherapie oder zur Differentialdiagnose von COPD, die Detektion von Lungentumoren, TBC oder Lungenentzündung.
Aus der EP 1384 069 ist ein Atemkonversionsmodul bekannt, dem das Atemgas zugeführt wird, bevor es zur eigentlichen Sensoreinheit geleitet wird. Die Aufgaben dieses Atemkonversionsmodul sind beispielsweise die Entfeuchtung des Atems, um eine zu starke Belastung der Sensoreinheit mit Feuchte zu vermeiden, sowie eine Umwandlung bestimmter Atemkomponenten. Im Fall der Asthmaanwendung wird z. B. hier NO zu NO₂ oxidiert. Dies ist ein Beispiel für die Konverterfunktion, bei der ein nur schwierig zu detektierendes Analytgas in ein gut vom Sensor zu messendes anderes Gas umgewandelt wird. In anderen Fällen werden Störgase zu nicht vom Sensor detektierbaren Gasen oxidiert.

Aus dem Dokument WO 2001/115694 A1 ist ebenfalls eine Vorrichtung zur Gasanalyse mit einem Konversionsmodul zur Umwandlung bestimmter Atemkomponenten bekannt, wobei dem Konversionsmodul dieser Vorrichtung eine Einrichtung zur Luftentfeuchtung vorgeschaltet ist.
Diese Konverter werden verbraucht und ihre Lebensdauer ist begrenzt. Es ist möglich, einen Austausch des Filters oder Konverters nach einer abgeschätzten Lebenszeit oder Anzahl von Benutzungszyklen vorzunehmen. Diese Methode ist jedoch störanfällig, da ein exaktes Monitoren der Benutzungsintensität notwendig ist. Auch andere Fehler sind möglich: So wird beispielsweise zur Vermeidung von Alterung im Lagerzustand der Filter oder Konverter per Verpackung vor Benutzung oder per Ventilsteuerung im Gerät von der Umgebungsluft abgeschottet. Wenn das nicht klappt, besteht die Gefahr vorzeitiger Alterung und von Fehlmessungen des Atemanalysators.
Es ist Aufgabe der vorliegenden Erfindung, eine Vorrichtung zur Gasanalyse eines Gasgemischs anzugeben, die angesichts der beschriebenen Probleme verbessert ist. Diese Aufgabe wird durch eine Vorrichtung mit den Merkmalen von Anspruch 1 gelöst.
Für die Erfindung wurde erkannt, dass eine direkte Messung der Funktion des Konverters über den Zielanalyten, beispielsweise NO, nicht möglich ist, da naturgemäß dessen Konzentration unbekannt ist. Daher wird eine indirekte Messung über eine gekoppelte Gaskonversion verwendet.
Die erfindungsgemäße Vorrichtung zur Gasanalyse eines Gasgemischs umfasst einen Gaskonverter zur Umwandlung einer ersten Gaskomponente in eine Zielgaskomponente, ein Sensorsystem zur Detektion der Zielgaskomponente oder einer anderen Komponente des Gasgemischs nach der Umwandlung durch den Gaskonverter. Weiterhin ist der Gaskonverter ausgestaltet, die Konzentration einer zweiten Gaskomponente zu verändern oder eine Umwandlung in eine zweite Zielgaskomponente zu bewirken und das Sensorsystem ist ausgestaltet, die Konzentration der zweiten Gaskomponente oder der zweiten Zielgaskomponente zu bestimmen. Schließlich ist eine Auswerteeinrichtung vorgesehen, ausgestaltet, anhand der Konzentration der zweiten Gaskomponente oder der zweiten Zielgaskomponente einen Wert für die Alterung des Gaskonverters zu ermitteln.
Ausgenutzt wird mit anderen Worten ein Konverter, der ein anderes, im Atemgas in einer charakteristisch anderen Konzentration als in Raumluft vorkommendes Gas umsetzt oder entfernt. Beispiele hierfür sind Wasser oder CO₂. Die Umsetzung dieses Gases wird dann mit einem zusätzlichen Sensor für dieses Gas, der hinter dem Konverter angebracht ist, gemessen.

Dabei wird die Konzentration dieses Gases beim Einblasen in das Gerät gemessen. Wenn die Konzentration dieses zweiten Gases (dessen Konzentration in der Ausatemluft bekannt und ausreichend stabil ist) hinlänglich verändert wird, ist der Konverter funktionstüchtig, andernfalls wird beispielsweise eine Alarmmeldung generiert.
Hierzu wird der Konverter zweckmäßig so gebaut, dass die Konversion des Hilfsgases korreliert mit der Konversion des Zielgases altert. Die beiden Alterungsverhalten können im Experiment bestimmt werden, so dass eine Auswertelogik dann aus der Alterung der Konversion des Hilfsgases auf die Alterung der Konversion des Zielgases schließen kann. Erstmals wird somit vorteilhaft eine Selbstüberwachung des Atemkonverters durchgeführt.
In einer Weiterbildung der Erfindung ist der Gaskonverter ausgestaltet, eine Speicherung der zweiten Gaskomponente zu bewirken. In diesem Fall wird die zweite Gaskomponente chemisch oder physikalisch gebunden.
Bevorzugt ist die Auswerteeinrichtung ausgestaltet, den Wert für die Alterung des Gaskonverters daran zu bestimmen, wann nach einem Gaszutritt vom Gasgemisch die Konzentration der zweiten Gaskomponente oder der zweiten Zielgaskomponente einen Schwellwert über- oder unterschreitet.
In einer alternativen Ausgestaltung oder zusätzlich ist die Auswerteeinrichtung ausgestaltet, den Wert für die Alterung des Gaskonverters anhand der Steigung der zweiten Gaskomponente oder der zweiten Zielgaskomponente einen Schwellwert zu bestimmen.
Ein bevorzugtes, jedoch keinesfalls einschränkendes Ausführungsbeispiel für die Erfindung wird nunmehr anhand der Figuren der Zeichnung näher erläutert. Dabei sind die Merkmale schematisiert dargestellt. Es zeigen
Figur 1 Messkurven von Sensoren eines Asthma-Atemanalysators,
Figur 2 den Aufbau des Asthma-Atemanalysators,
Figur 3 den Aufbau eines Konverters.

Die Figuren zeigen ein Beispiel für ein Messgerät 10 zur Stickoxidmessung im menschlichen Atem, einen Asthmasensor.

Hier wird der Atem über einen Konverter 11 zugeführt, sowie über ein System von Ventilen 15, 16, 17, 19 dafür gesorgt, dass entweder konvertierte Atemluft oder gereinigte Frischluft zur Regenerierung der Sensorgrundlinie der sich in einer Messkammer 18 befindlichen Sensoren zugeführt wird.

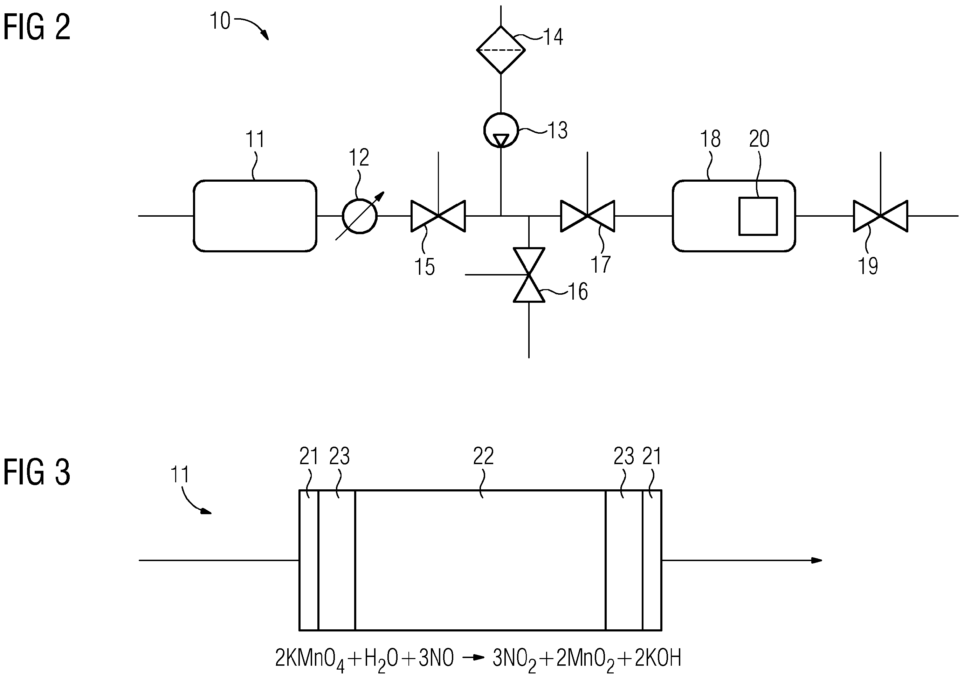

Das beispielhafte Messgerät 10 ist dabei wie in Figur 2 dargestellt aufgebaut. Dem Konverter 11 ist Atemluft zuführbar. Der Konverter 11 ist mit einer gasführenden Leitung mit einem Flussmessgerät 12 verbunden. Darauf folgt ein Einlassventil 15. Auf das Einlassventil 15 folgt ein Knotenpunkt der Gasleitung. An dem Knotenpunkt verzweigt die gasführende Leitung. In einem ersten Ast sind vom Knotenpunkt aus gesehen eine Pumpe 13 und darauf folgend ein Filter 14 für Referenzluft mit Aktivkohle vorgesehen. Der erste Ast ermöglicht die Zuführung gereinigter Frischluft zur Regenerierung der Sensorgrundlinie zur Messkammer 18 mit einem Sensorsystem 20. In einem zweiten Ast ist vom Knotenpunkt aus gesehen ein Auslassventil 16 vorgesehen. Ein dritter Ast beinhaltet vom Knotenpunkt aus gesehen ein zweites Einlassventil 17, die Messkammer 18 und ein zweites Auslassventil 19.

Der Konverter 11 hat hier zweierlei Aufgaben. Er besteht wie in Figur 3 dargestellt aus Gittern 21 an beiden Enden, zwischen denen Filterelemente 23 angeordnet sind. Der Wandler 22 ist als Keramik mit Kaliumpermanganat KMnO₄ ausgeführt, genauer als Silikagel, das zum Teil mit KMnO₄ imprägniert ist. Die Hauptfunktion hier ist die Umwandlung von Stickstoffmonoxid NO der Ausatemluft in mit dem Sensor messbares Stickstoffdioxid NO₂, was durch das KMnO₄ bewirkt wird. Die zweite Funktion ist die Entfernung überschüssiger Atemfeuchte durch das Silikagel. Bei diesem Aufbau besteht eine Korrelation der Alterung beider Funktionen.

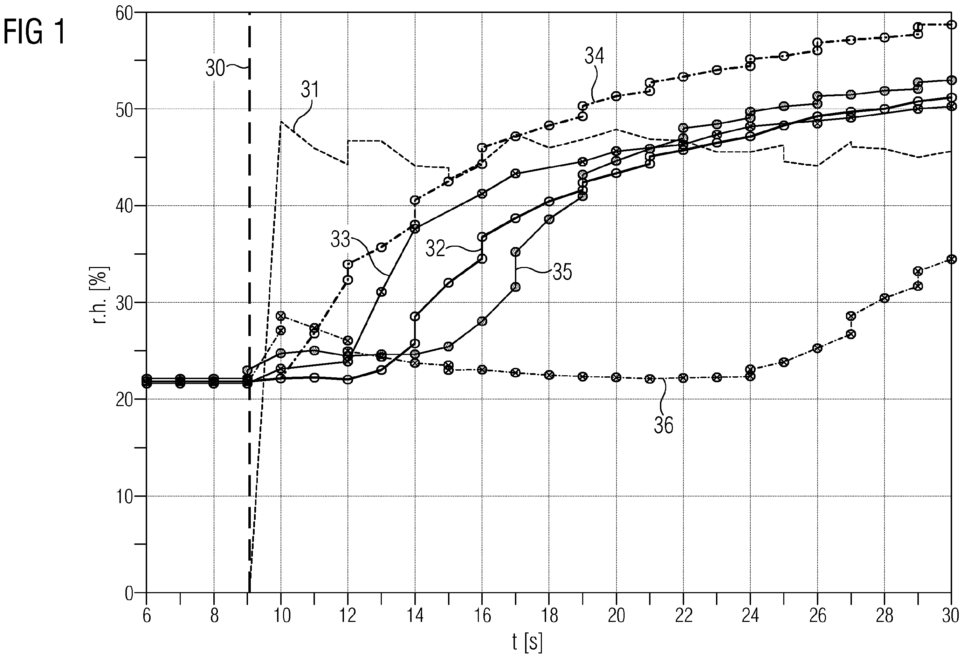

Figur 1 zeigt die Funktion der indirekten Messung anhand der relativen Feuchte. In Figur 1 ist die relative Feuchte nach dem Konverter 11 gegen die Zeit in Sekunden aufgetragen für fünf verschiedene Proben. Die relative Feuchte wird dabei mit einem Sensor in der Messkammer 18 gemessen. Zu einem Zeitpunkt 30 wird dabei neue Ausatemluft mit relativ hohem Feuchtegehalt eingeblasen.

Eine erste Messkurve 36 zeigt den Verlauf der relativen Feuchte bei einem neuen Konverter 11. Hier steigt die relative Feuchte nach dem Konverter 11 fast nicht an. Eine zweite Messkurve 32 und einer dritte Messkurve 35 zeigen den Verlauf der relativen Feuchte bei einem leicht gealterten Konverter 11. Hier steigt die relative Feuchte nach dem Konverter 11 nach einer Zeit von 8-10 Sekunden nach dem Zugeben der Ausatemluft an. Eine vierte und fünfte Messkurve 33, 34 zeigen den Verlauf der Feuchte nach dem Konverter 11 bei stark gealterten Konvertern 11. Bei diesen steigt die Feuchte direkt nach dem Zugeben der Ausatemluft steil an.

Aus der Verzögerung, mit der die Feuchte nach dem Konverter 11 ansteigt und/oder aus der Steilheit des Anstiegs gewinnt eine Auswerteeinrichtung, die in Figur 2 nicht dargestellt ist, einen Wert für die Alterung des Konverters 11. Dabei ist zu beachten, dass die eigentliche Messaufgabe, d.h. die Messung von NO bzw. NO₂ hierzu nicht verwendet wird, da die Konzentration des NO ja unbekannt ist und somit die Effektivität des Konverters somit anhand der gemessenen Konzentration von NO₂ nicht abgeschätzt werden kann. Die Konzentration der Feuchte in der Ausatemluft hingegen ist relativ gut bekannt.

Ausgegangen wird also von Umgebungsluft mit relativ geringer Feuchte. Wenn nun Atem durch einen neuen Konverter eingeblasen wird, findet nur ein geringfügiger Anstieg der Gasfeuchte statt, während der Anstieg immer stärker ausfällt bei gealterten Konvertern. Daher kann aus dem Verlauf des Anstiegs der Gasfeuchte auf den Alterungszustand des Konverters geschlossen werden. In Figur 1 zusätzlich noch dargestellt ist der Ausatemdruck 31, der den Einblasvorgang charakterisiert.

## Patentansprüche

1. Vorrichtung (10) zur Gasanalyse eines Gasgemischs mit:
- einem Konverter (11) zur Umwandlung einer ersten Gaskomponente in eine Zielgaskomponente,
- einem Sensorsystem (20) zur Detektion der Zielgaskomponente oder einer anderen Komponente des Gasgemischs nach der Umwandlung durch den Konverter (11), **dadurch gekennzeichnet, dass**
- der Konverter (11) ausgestaltet ist, die Konzentration einer zweiten Gaskomponente zu verändern oder eine Umwandlung in einer zweiten Zielgaskomponente zu bewirken,
- das Sensorsystem (20) ausgestaltet ist, die Konzentration der zweiten Gaskomponente oder der zweiten Zielgaskomponente zu bestimmen,
- eine Auswerteeinrichtung vorgesehen ist, ausgestaltet, anhand der Konzentration der zweiten Gaskomponente oder der zweiten Zielgaskomponente einen Wert für die Alterung des Konverters (11) zu ermitteln.

2. Vorrichtung (10) nach Anspruch 1, bei der der Konverter (11) eine Umwandlung der zweiten Gaskomponente in eine zweite Zielgaskomponente vornimmt.

3. Vorrichtung (10) nach Anspruch 1 oder 2, bei der der Konverter (11) ausgestaltet ist, eine Speicherung der zweiten Gaskomponente zu bewirken.

4. Vorrichtung (10) nach einem der vorangehenden Ansprüche, bei der die erste Gaskomponente Stickstoffmonoxid ist und die erste Zielgaskomponente Stickstoffdioxid.

5. Vorrichtung (10) nach einem der vorangehenden Ansprüche, bei der die Auswerteeinrichtung ausgestaltet ist, den Wert für die Alterung des Konverters (11) daran zu bestimmen, wann nach einem Zutritt vom Gasgemisch die Konzentration der zweiten Gaskomponente oder der zweiten Zielgaskomponente einen Schwellwert über- oder unterschreitet.

6. Vorrichtung (10) nach einem der vorangehenden Ansprüche, bei der die Auswerteeinrichtung ausgestaltet ist, den Wert für die Alterung des Konverters (11) anhand der Steigung der zweiten Gaskomponente oder der zweiten Zielgaskomponente zu bestimmen.

7. Verwendung einer Vorrichtung nach einem der Ansprüche 1 bis 6 zur Asthma-Atemanalyse.

## Claims

1. Apparatus (10) for the gas analysis of a gas mixture, having:
- a converter (11) for conversion of a first gas component into a target gas component, and
- a sensor system (20) for detection of the target gas component or of another component of the gas mixture after the conversion by the converter (11), **characterized in that**
- the converter (11) is configured in order to modify the concentration of a second gas component or to cause conversion into a second target gas component,
- the sensor system (20) is configured in order to determine the concentration of the second gas component or of the second target gas component, and
- an evaluation device is provided, which is configured in order to ascertain a value of the ageing of the converter (11) with the aid of the concentration of the second gas component or of the second target gas component.

2. Apparatus (10) according to Claim 1, wherein the converter (11) carries out conversion of the second gas component into a second target gas component.

3. Apparatus (10) according to Claim 1 or 2, wherein the converter (11) is configured in order to cause storage of the second gas component.

4. Apparatus (10) according to one of the preceding claims, wherein the first gas component is nitrogen monoxide and the first target gas component is nitrogen dioxide.

5. Apparatus (10) according to one of the preceding claims, wherein the evaluation device is configured in order to determine the value of the ageing of the converter (11) from the fact that, when after the gas mixture enters, the concentration of the second gas component or of the second target gas component is greater or less than a threshold value.

6. Apparatus -(10) according to one of the preceding claims, wherein the evaluation device is configured in order to determine the value of the ageing of the converter (11) with the aid of the gradient of the second gas component or of the second target gas component.

7. Use of an apparatus according to one of Claims 1 to 6 for asthma breath analysis.

## Revendications

1. Dispositif (10) pour l'analyse de gaz d'un mélange gazeux avec:
- un convertisseur (11) pour la conversion d'un premier composant gazeux en un composant de gaz cible,
- un système de capteur (20) pour la détection du composant de gaz cible ou d'un autre composant du mélange gazeux après la conversion par le convertisseur (11), **caractérisé en ce que**
- le convertisseur (11) est configuré pour modifier la concentration d'un deuxième composant gazeux ou pour opérer une conversion en un deuxième composant de gaz cible,
- le système de capteur (20) est configuré pour déterminer la concentration du deuxième composant gazeux ou du deuxième composant de gaz cible,
- il est prévu un dispositif d'évaluation, configuré pour déterminer, à l'aide de la concentration du deuxième composant gazeux ou du deuxième composant de gaz cible, une valeur exprimant le vieillissement du convertisseur (11) .

2. Dispositif (10) selon la revendication 1, dans lequel le convertisseur (11) opère une conversion du deuxième composant gazeux en un deuxième composant de gaz cible.

3. Dispositif (10) selon la revendication 1 ou 2, dans lequel le convertisseur (11) est configuré pour provoquer une accumulation du deuxième composant gazeux.

4. Dispositif (10) selon l'une quelconque des revendications précédentes, **caractérisé en ce que** le premier composant gazeux est le monoxyde d'azote et le premier composant de gaz cible est le dioxyde d'azote.

5. Dispositif (10) selon l'une quelconque des revendications précédentes, dans lequel le dispositif d'évaluation est configuré pour déterminer la valeur exprimant le vieillissement du convertisseur (11) à l'instant où, après une entrée de mélange gazeux, la concentration du deuxième composant gazeux ou du deuxième composant de gaz cible devient plus élevée ou plus basse qu'une valeur de seuil.

6. Dispositif (10) selon l'une quelconque des revendications précédentes, dans lequel le dispositif d'évaluation est configuré pour déterminer la valeur exprimant le vieillissement du convertisseur (11) à l'aide de l'augmentation du deuxième composant gazeux ou du deuxième composant de gaz cible.

7. Utilisation d'un dispositif selon l'une quelconque des revendications 1 à 6 pour l'analyse d'haleine d'asthme.
